(19) **Europäisches Patentamt**
**European Patent Office**
**Office européen des brevets**

(11) **EP 2 440 149 B1**

(12) **EUROPÄISCHE PATENTSCHRIFT**

(45) Veröffentlichungstag und Bekanntmachung des Hinweises auf die Patenterteilung:
**14.08.2013 Patentblatt 2013/33**

(21) Anmeldenummer: **10726431.9**

(22) Anmeldetag: **09.06.2010**

(51) Int Cl.:
***A61B 18/12*** *(2006.01)*

(86) Internationale Anmeldenummer:
**PCT/EP2010/003467**

(87) Internationale Veröffentlichungsnummer:
**WO 2010/142438 (16.12.2010 Gazette 2010/50)**

(54) **VERSORGUNGSEINRICHTUNG ZUR BEREITSTELLUNG EINER HF-AUSGANGSSPANNUNG, HF-CHIRURGIEGERÄT MIT ENTSPRECHENDER VERSORGUNGSEINRICHTUNG**

SUPPLY DEVICE FOR PROVIDING AN HF OUTPUT VOLTAGE, HF SURGICAL INSTRUMENT COMPRISING A CORRESPONDING SUPPLY DEVICE

DISPOSITIF D'ALIMENTATION DESTINÉ À FOURNIR UNE TENSION DE SORTIE HF, APPAREIL CHIRURGICAL HF POURVU D'UN DISPOSITIF D'ALIMENTATION CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **10.06.2009 DE 102009024612**

(43) Veröffentlichungstag der Anmeldung:
**18.04.2012 Patentblatt 2012/16**

(73) Patentinhaber: **ERBE Elektromedizin GmbH**
**72072 Tübingen (DE)**

(72) Erfinder:
• **SCHALL, Heiko**
  **72622 Nürtingen (DE)**
• **FRITZ, Martin**
  **72070 Tübingen (DE)**

(74) Vertreter: **Rüger, Barthelt & Abel**
**Webergasse 3**
**73728 Esslingen (DE)**

(56) Entgegenhaltungen:
**WO-A1-2009/015278     WO-A2-2007/092610**
**WO-A2-2008/022148     US-A1- 2007 197 891**
**US-A1- 2008 091 193**

## Beschreibung

Die Erfindung betrifft eine Versorgungseinrichtung zur Bereitstellung einer HF-Ausgangsspannung, ein HF-Chirurgiegerät mit einer entsprechenden Versorgungseinrichtung und ein Verfahren zum Betreiben einer HF-Generatoreinheit für ein elektrochirurgisches Instrument.

In der Hochfrequenzchirurgie (HF-Chirurgie) wird Wechselstrom mit hoher Frequenz durch den menschlichen Körper geleitet, um Gewebe gezielt zu schädigen bzw. zu schneiden oder zu koagulieren. Hierbei wird allgemein zwischen einer monopolaren und einer bipolaren Applikation des Behandlungsstroms unterschieden. Bei einer monopolaren Anwendung ist üblicherweise nur eine Applikationselektrode vorgesehen, an die eine hochfrequente Wechselspannung angelegt wird. Die Applikationselektrode befindet sich beispielsweise an einem elektrochirurgischen Instrument zum Schneiden und/oder Koagulieren von Gewebe. Ferner wird eine großflächige Neutralelektrode am Körper des Patienten angebracht. Bei der Applikation des HF-Stroms wird eine HF- Spannung an die Applikationselektrode und die Neutralelektrode angelegt, wobei der Stromkreis über das dazwischen liegende Gewebe geschlossen wird. Die Form der Applikationselektrode hängt von dem jeweiligen Anwendungsgebiet ab. Die Oberflächen oder Kontaktflächen der Applikationselektrode, über die der Wechselstrom in das Gewebe geleitet wird, ist verhältnismäßig klein, so dass in der direkten Umgebung der Applikationselektrode eine hohe Stromdichte und demzufolge auch eine hohe Wärmeentwicklung entstehen.

Die Stromdichte nimmt mit zunehmendem Abstand zur Applikationselektrodc stark ab, sofern nicht durch erhebliche Unterschiede in der Gewebeleitfähigkeit auch an weiteren Körperstellen hohe Stromdichten auftreten. Bei bipolaren Anwendungen wird die HF-Spannung an zwei nahe beieinander liegenden Applikationselektroden, z.B. an einer bipolaren Koagulationszange angelegt. Das heißt, der Strom fließt hauptsächlich durch zwischen den Applikationselektroden befindliches Gewebe. Vorzugsweise sind die Applikationselektroden an einem Instrument angeordnet. Schlecht zugängliche Gewebestellen können oftmals nur schlecht oder gar nicht mit solchen Instrumenten erreicht und behandelt werden.

Versorgungseinrichtungen versorgen die Instrumente mit einer geeigneten HF-Spannung und einem geeigneten HF-Strom, so dass eine problemlose Behandlung des Patienten möglich ist. HF-Strom und HF-Spannung können in Abhängigkeit von dem zu behandelnden Gewebe sowie von dem verwendeten Betriebsmodus (z.B. Koagulationsmodus, Schneidmodus) variieren. Weitere Betriebsmodi für unterschiedliche Gewebetypen sind denkbar. Die Versorgungseinrichtung muss eine ständige Regelung der HF-Ausgangsspannung und/oder des HF-Ausgangsstroms vornehmen, um einen geeigneten Betrieb des elektrochirurgischen Instruments sicherzustellen. Eine Anpassung der genannten Größen ist mitunter deshalb notwendig, da sich die Beschaffenheit des Gewebes, das mittels eines Instruments behandelt wird, ständig ändert. So nimmt beispielsweise wegen des applizierten HF-Stroms die Leitfähigkeit des Gewebes ab und somit der Widerstand zu. Andererseits haben auch unbehandelte Gewebetypen (z.B. Fettgewebe, Muskelgewebe, Nervengewebe) unterschiedliche Widerstände, die es bei einer Spannungsregelung oder einer Stromregelung zu berücksichtigen gilt.

Üblicherweise bemüht man sich, eine derartige Regelung bereitzustellen, dass der behandelnde Arzt unabhängig von dem Gewebetyp oder dessen Beschaffenheit ein sicheres Schneiden und/oder Koagulieren bewerkstelligen kann. Um also die Funktion des elektrochirurgischen Instruments sicherzustellen, muss die Versorgungseinrichtung die Leistung des HF-Generators oder der HF-Generatoreinheit steuern bzw. regeln. Die hier bereitgestellte Leistung wird üblicherweise durch die Leistungsfähigkeit des verwendeten Generators und entsprechende Sicherheitsrichtlinien beschränkt. So kann es vorkommen, dass mit einer sehr hohen Leistung behandelt wird, ohne dass dies von dem Arzt wahrgenommen wird. Dies ist gefährlich und kann zu einer ungewollten Schädigung von Gewebe führen. Des Weiteren ist die Handhabung eines entsprechenden Instruments für den Arzt ungewohnt, da er beispielsweise bei mechanischen Schneidvorgängen an eine haptischc Rückmeldung gewohnt ist.

Aus der WO 2009/015278 ist eine Versorgungseinrichtung zur Bereitstellung einer HF-Ausgangsspannung zur Applikation mittels eines chirurgischen Instruments bekannt. Diese Einrichtung umfasst eine HF-Generatoreinheit zur Erzeugung der HF-Ausgangsspannung, eine Messeinheit zur Bestimmung einer elektrischen Last während der Applikation eines HF-Ausgangsstroms und eine Signaleinheit zur Ausgabe eines haptisch wahrnehmbaren Signals.

Ausgehend von diesem Stand der Technik ist es Aufgabe der vorliegenden Erfindung, eine Versorgungseinrichtung der eingangs genannten Art bereitzustellen, die eine sichere und funktionale Bedienung ermöglicht und den Umgang mit einem entsprechenden elektrochirurgischen Instrument erleichtert. Des Weiteren soll ein entsprechendes HF- Chirurgiegerät, ein Verfahren zum Betreiben einer HF-Generatoreinheit und ein Verfahren zur Durchführung eines elektrochirurgischen Eingriffs angegeben werden.

Diese Aufgabe wird erfindungsgemäß durch eine Versorgungseinrichtung gemäß dem Anspruch 1 gelöst.

Insbesondere wird die Aufgabe durch eine Versorgungseinrichtung zur Bereitstellung einer HF-Ausgangsspannung zur Applikation mittels eines elektrochirurgischen Instruments gelöst, wobei die Versorgungseinrichtung umfasst:

- eine HF-Gcneratoreinheit zur Erzeugung der HF-Ausgangsspannung;
- eine Messeinheit zur Bestimmung einer elektrischen Last während der Applikation eines HF-Ausgangsstroms;
- eine Signaleinheit zur Ausgabe eines haptisch wahrnehmbaren Signals; und
- eine Steuereinheit, die die Messeinheit und die Signaleinheit umfasst und ausgebildet ist, um die Signaleinheit in Abhängigkeit von der elektrischen Last zu aktivieren.

Ein zentraler Gedanke der vorliegenden Erfindung besteht also darin, ein haptisch wahrnehmbares Signal, insbesondere am Instrument, bereitzustellen, das angibt, mit welcher Last die HF-Generatoreinheit zum aktuellen Zeitpunkt betrieben wird. Damit wird dem Chirurgen bzw. Arzt eine haptische Rückmeldung über die verschiedenen Schnittparameter wie beispielsweise Schnittgeschwindigkeit und/oder Schnitttiefe und/oder Gewebeart gegeben. Die Signaleinheit und/oder die Messeinheit können als logische Bestandteile der Steuereinheit verstanden werden, die die gegebene Funktion implementieren. Somit kann die Signaleinheit auch ein Bestandteil eine zur Reglung der HF-Ausgangsspannung verwendeten Reglers sein.

Die Signaleinheit kann zur Ausgabe des Signals auf die HF-Generatoreinheit einwirken, um die HF-Ausgangsspannung und/oder den HF-Ausgangsstrom zu reduzieren. Somit kann ein Effekt bereitgestellt werden, der beispielsweise während eines Schnitts die Spannung und/oder den Strom am Applikator oder der Applikationselektrode - je nach elektrischer Last des Gewebes - einbrechen lässt. Somit kann dem behandelnden Arzt ein Gefühl für die applizierte Leistung oder die vorhandene Last mittels des Instruments vermittelt werden. Der Umgang mit dem Instrument gestaltet sich ähnlich wie die Bedienung von herkömmlichen mechanischen Instrumenten. So spürt der Arzt beispielsweise beim tiefen Eindringen eines Instruments in das Gewebe einen mechanischen Widerstand. Entsprechend höher kann auch der gefühlte Widerstand sein, wenn ein schneller Schnitt mittels des elektrochirurgischen Instruments durchgeführt wird. Es ist möglich, den mechanischen und den elektrischen Widerstand in Bezug zu setzen. Ein Aktivieren der Signaleinheit kann beispielsweise darin gesehen werden, dass die HF-Ausgangsspannung so geregelt wird, dass beim Durchführen eines Schneidvorgangs in einem Gewebe die molekulare Auflösung des Gewebes derart langsam eintritt, dass die Bewegung des Instruments beim Schneidvorgang durch einen spürbaren mechanischen Kontakt zu dem Gewebe gebremst wird.

Die Messeinheit kann eine Wirkleistungsberecheneinheit zur Berechnung einer Wirkleistung und die Steuereinheit ein, insbesondere einstellbares, Widerstandselement umfassen, um einen gemäß der Wirkleistung angepassten Sollwert für die HF-Generatoreinheit vorzugeben. Vorzugsweise wird also die Wirkleistungsbercheneinheit verwendet, um eine Wirkleistung des applizierten HF-Stroms zu bestimmen oder abzuschätzen. Die Steuereinheit nutzt entsprechende Messsignale, um Sollwerte für die HF-Generatoreinheit vorzugeben. Diese Sollwerte können auch Steuersignale sein, die den Betrieb der HF-Generatoreinheit steuern bzw. regeln. Somit kann über die Sollwertvorgabe die HF-Ausgangsspannung und/oder der HF-Ausgangsstrom eingestellt werden.

Das Widerstandselement kann einen einstellbaren Widerstand haben. Somit ist es beispielsweise möglich, das haptisch wahrnehmbare Signal über die Zeit zu variieren. So kann die HF-Ausgangsspannung beim Auftreten einer hohen Last kurzfristig einbrechen, so dass es zu einer deutlichen Rückmeldung kommt. Im weiteren Verlauf eines Schnittes kann die HF-Ausgangsspannung wieder derart angehoben werden, dass der gefühlte mechanische Widerstand vergleichsweise gering ist. Alternativ können mittels des einstellbaren Widerstands der Versorgungseinrichtung innewohnende Innenwiderstände und Widerstände an Versorgungsleitungen für das Instrument kompensiert werden. Allgemein kann das Widerstandselement dazu verwendet werden die Ausprägung des Einbruchs der HF-Ausgangsspannung zu parametrisieren. Es ist möglich den gefühlten mechanischen Widerstand bei einem Schneidvorgang und somit das haptisch wahrnehmbare Signal anhand des Widerstandselements zu verändern.

Die Versorgungseinrichtung kann eine Einstellvorrichtung zum Einstellen eines Sollwerts, insbesondere einer Sollausgangsspannung oder eines Sollausgangsstroms oder einer Sollleistung der Versorgungseinrichtung, sowie eine Regeleinheit zum Regeln der HF-Generatoreinheit gemäß dem Sollwert umfassen. Der Betrieb der Generatoreinheit kann also, wie eingangs erwähnt, mittels eines Regelkreislaufs gesteuert oder geregelt werden. Hierbei ist sowohl eine Spannungsregelung als auch eine Stromregelung denkbar. Die gemessene Last oder die gemessene Wirkleistung können dazu verwendet werden, den Sollwert eines entsprechenden Regelkreislaufs anzupassen. Es kann sich also beispielsweise um eine unterlagerte Spannungsregelung handeln.

Vorzugsweise handelt es sich bei der Versorgungseinrichtung um eine Versorgungseinrichtung zum Betreiben der HF-Generatoreinheit in einer spannungsgeregelten Betriebsart. Bei der spannungsgeregelten Betriebsart versucht man, die vom Leistungsoszillator bzw. von der HF-Generatoreinrichtung abgegebene HF-Spannung bzw. HF-Ausgangsspannung konstant zu halten. Im Verlauf eines mit dem Instrument durchgeführten Schnittes variiert die Spannung des an die HF-Generatoreinrichtung angeschlossenen Leistungsnetzteil, da es regelungstechnisch nun die Funktion eines Stellglieds übernimmt. Bei der üblichen spannungsgeregelten Betriebsart bricht die HF-Ausgangsspannung bei einer Gewebeimpedanzänderung nicht ein. Es ist eine Intension der vorliegenden Erfindung, das Verhalten einer ungeregelten Betriebsart - bei der die HF-Ausgangsspannung in Abhängigkeit von der Gewebeimpedanz variiert - der geregelten Betriebsart kontrolliert aufzuprägen. Hierbei soll die Ausprägung der Reaktion parametrisierbar und somit kontrollierbar sein. Diese Aufgabe wird mittels einer Innenwiderstandssimulation bewerkstelligt. Das heißt, das System wird in der spannungsgeregelten Betriebsart betrieben, wobei eine Einstellvorrichtung zum Einstellen eines Sollwerts und eine Regeleinheit zum Regeln der HF-Gcneratoreinheit gemäß dem Sollwert vorgesehen ist. Die Versorgungseinrichtung bestimmt vorzugsweise den Sollwert unter Berücksichtigung des HF-Ausgangsstroms, insbesondere anhand eines Vorgabewerts und des HF-Ausgangsstroms.

Eine Komparatoreinheit kann vorgesehen sein, um den Sollwert zu bestimmen, wobei vorzugsweise der Sollwert anhand der Formel $U_{Soll1}=U_0-R_1 * I_3$ ist. Somit wird bei der Bestimmung des Sollwerts der HF-Ausgangsstrom und damit die Gewebeimpedanz berücksichtigt.

Die genannte Reglereinheit für die HF-Ausgangsspannung kann einen PI-Regler umfassen.

Die eingangs genannte Aufgabe wird des Weiteren durch ein HF-Chirurgiegerät mit einer Versorgungseinrichtung gelöst, wobei die Versorgungseinrichtung vorzugsweise wie vorab beschrieben ausgebildet ist und eine Innenwiderstandseinstellvorrichtung zur Einstellung eines Innenwiderstands, insbesondere gemäß einem Versorgungskabelwiderstands für das elektrochirurgische Instrument hat. Bei herkömmlichen Geräten lässt sich der Innenwiderstand nicht einstellen. Erfindungsgemäß soll ein einstellbarer Innenwiderstand bereitgestellt werden, der in sich wieder entsprechend der Wirkleistung bzw. des Wirkstroms der HF-Generatoreinheit eingestellt wird. Somit ist es auch möglich, weitere Widerstände, beispielsweise einen Kabelwiderstand zu kompensieren.

Das HF-Chirurgiegerät kann ein elektrisches Skalpell oder dergleichen schneidende Instrumente umfassen, die an die Versorgungseinrichtung angeschlossen sind.

Des Weiteren wird die Aufgabe durch ein Verfahren zum Betreiben einer HF-Generatoreinheit gemäß dem Anspruch 12 gelöst.

Insbesondere wird die Aufgabe durch ein Verfahren zum Betreiben einer HF-Generatoreinheit für mindestens ein elektrochirurgisches Instrument gelöst, wobei das Verfahren die folgenden Schritte umfasst:

- Regeln einer HF-Ausgangsspannung gemäß einem Stellwert mittels eines Spanungreglers;
- Erfassen einer elektrischen Last der HF-Generatoreinheit;
- Korrigieren des Stellwerts in Abhängigkeit von der erfassten Last.

Die Aufgabe wird des Weiteren durch ein Verfahren zur Durchführung eines elektrochirurgischen Eingriffs gelöst, wobei das Verfahren die folgenden Schritte umfasst:

- Einstellen eines Vorgabewerts, um anzugeben, mit welcher Leistung oder Spannung ein elektrochirurgisches Instrument betrieben werden soll,
- Betreiben einer HF-Generatoreinheit, wobei die HF-Generatoreinheit derart in einer spannungsgeregelten Betriebsart betrieben wird, dass eine HF- Ausgangsspannung im Wesentlichen konstant gemäß einem Sollwert auf ein Gewebe appliziert wird,
- Messen eines HF-Ausgangsstroms,
- Verringern des Sollwerts, wenn der gemessene HF-Ausgangsstrom ansteigt, um der den Eingriff durchführenden Person zu signalisieren, dass die Impedanz des Gewebes zunimmt.

Die den Eingriff durchführende Person erhält also eine haptisch wahrnehmbare Rückmeldung, die es ihr ermöglicht, die vorliegende Last und/oder die aufgebrachte Leistung einzuschätzen. Dies ist bei herkömmlichen spannungsgeregelten HF- Generatoreinheiten nicht möglich.

Dementsprechend kann der Sollwert erhöht werden, wenn der gemessene HF- Ausgangsstrom ansteigt, um der den Eingriff durchführenden Person zu signalisieren, dass die Impedanz des Gewebes abnimmt.

Das Instrument kann in einer Schneidbewegung durch das Gewebe geführt werden und die HF-Generatoreinheit derart betrieben werden, dass die für die Schneidbewegung aufzuwendende Kraft in Abhängigkeit von der Gewebeimpedanz variiert. Bei einer Regelung des HF-Ausgangsstroms derart, dass dieser geringfügig niedriger ist als der gewünschte HF-Ausgangsstrom, wird das elektrochirurgische Instrument mit einem gewissen mechanischen Widerstand zurückgehalten (verursacht durch einen Gewebekontakt mit Gewebe, das noch nicht durchtrennt wurde). Der Schneidvorgang geht also wesentlich schwerer. Auf der anderen Seite führt ein Anlegen einer, hohen HF-Ausgangsspannung dazu, dass ein sehr geringer mechanischer Widerstand wirkt, so dass ein schnelles und einfaches Schneiden gewährleistet ist.

Für die genannten Verfahren ergeben sich Vorteile, die bereits in Bezug auf die Vorrichtung genannt wurden. Weitere vorteilhafte Ausführungsformen ergeben sich anhand der Unteransprüche.

Nachfolgend wird die Erfindung anhand von einigen Ausführungsbeispielen beschrieben, die mittels Abbildungen näher erläutert werden. Hierbei zeigen:

- Fig. 1 eine Versorgungseinrichtung, an die ein monopolares Instrument angeschlossen ist;
- Fig. 2 eine schematische Darstellung einer Steuereinheit der Versorgungseinrichtung mit Ein- und Ausgängen; und
- Fig. 3 einen Signalflussplan zur Steuerung bzw. Regelung der HF-Ausgangsspannung der Versorgungseinrichtung gemäß Fig. 1. In der nachfolgenden Beschreibung werden für gleiche und gleich wirkende Teile dieselben Bezugsziffern verwendet.

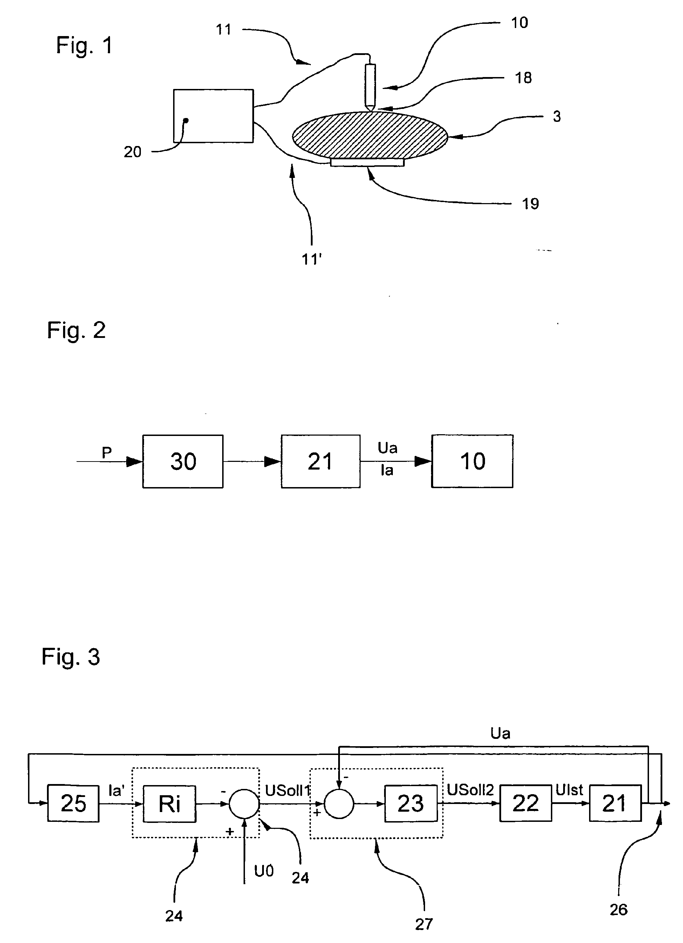

Fig. 1 zeigt ein elektrochirurgisches Gerät, umfassend eine Versorgungseinrichtung 20, ein monopolares elektrochirurgisches Instrument 10 und eine Neutralelektrode 19. Die Neutralelektrode 19 sowie das Instrument 10 sind über ein Kabel mit entsprechenden Leitungen 11, 11' an die Versorgungseinrichtung 20 angeschlossen. Die Versorgungseinrichtung 20 hat eine Steuereinheit 30 (vgl. Fig. 2), die unter anderem dafür zuständig ist, dass von einem vorhandenen HF-Generator 21 eine geeignete HF- Ausgangsspannung U3 und ein geeigneter HF-Ausgangsstrom $I_3$ zwischen der Neutralelektrode 19 und einer Applikationselektrode 18 des elektrochirurgischen Instruments 10 bereitgestellt wird. Hierfür steht der HF-Generator 21 und die Steuereinheit 30 in kommunikativer Verbindung. Die HF-Ausgangsspannung $U_3$ führt zu einer teilweisen Koagulation des Gewebes eines Torso 3.

Wie in Fig. 2 verdeutlicht, besteht ein Punkt der vorliegenden Erfindung darin, dass die Steuereinheit 30 eine Wirkleistung P des applizierten HF-Ausgangsstroms $I_3$ erfasst und so auf den HF-Generator 21 einwirkt, dass die Wirkleistung P - HF-Ausgangsspannung $U_a$ und HF-Ausgangsstrom I3 - eingestellt wird. Die HF-Ausgangsspannung Ua und der HF-Ausgangsstrom $I_3$ können derart gewählt sein, dass für die mit dem Instrument 10 vorzunehmende Tätigkeit, z.B. das Schneiden von Muskelgewebe, nur eine verhältnismäßig geringe Leistung zur Verfügung steht. Somit spürt der behandelnde Arzt bzw. Chirurg beim Führen des elektrochirurgischen Instruments 10 einen deutlichen mechanischen Widerstand. Das elektrochirurgische Instrument 10 schneidet zwar - ist also so mit einer HF-Ausgangsspannung U3 versorgt, dass eine ausreichende Leistung bereitgestellt wird -, jedoch gibt es eine haptische Rückmeldung.

Die Fig. 3 zeigt eine detailliertere Ausführung der erfindungsgemäßen Steuereinheit 30. Zur Einstellung einer geeigneten HF-Ausgangsspannung $U_3$ erfolgt eine Spannungsregelung, wobei ein unterlagerter Spannungsregler 27 die HF-Ausgangsspannung $U_3$, die durch den HF-Generator 21 erzeugt wurde, empfängt und mit einem Stellwert $U_{Soll1}$ vergleicht, den Differenzwert in einen PI-Regler 23 eingibt und so eine Netzteil-Sollspannung $U_{Soll2}$ generiert. Diese Netzteil-Sollspannung $U_{Soll2}$ wird in ein Netzteil 22 eingegeben, das eine Netzteil-Istspannung $U_{ist}$ bereitstellt und damit den HF-Generator 21 versorgt, der wiederum die HF-Ausgangsspannung $U_3$ und den HF-Ausgangsstrom $I_3$ generiert. Es erfolgt also eine Regelung der bereitgestellten Leistung anhand der HF-Ausgangsspannung $U_a$.

Die Vorrichtung der Fig. 3 erfasst eine Wirkleistung P mittels einer Wirkstromberecheneinheit 25. Diese ist an den HF-Ausgang angeschlossen und empfängt die HF- Ausgangsspannung $U_3$ und den HF-Ausgangsstrom $I_a$ und ermittelt einen Wirkstrom $I_{3'}$. Der Wirkstom $I_{a'}$ wird an einen Widerstand $R_i$ angelegt und die abfallende Spannung mit einer parametrisierbaren HF-Leerlaufspannung $W_0$ verglichen. Von der HF- Leerlaufspannung $U_0$ wird also die an dem Widerstand $R_j$ abfallende Spannung abgezogen, so dass sich der Stellwert $U_{Soll1}$ ergibt. Der Widerstand R und der Komparator 29 bilden also einen $R_i$-Simulator 24, der im Endeffekt einen Innenwiderstand des HF- Generators 21 simuliert. Der tatsächliche Innenwiderstand des HF-Generators 21 ist von aussen nicht mehr ersichtlich, da er durch den parametrisierbaren Widerstand $R_j$ kompensiert wird. Der $R_i$-Simulator 24 kann die Funktion einer Signaleinheit zur Ausgabe eines haptisch wahrnehmbaren Signals übernehmen.

Für den Stellwert $U_{Soll1}$ bzw. für die Sollspannung ergibt sich also die folgende Beziehung:

$$U_{Soll1} = U_0 - R_i * I_{a'},$$

wobei vorzugsweise $I_{3'}$ der Wirkanteil des ausgegebenen HF-Ausgangsstroms Ia ist. Der Stellwert $U_{Soll1}$ kann mittels des Komparators 29 berechnet werden.

In analoger Weise lässt sich auch ein Stromregelungssystem mit variierendem Innenleitwert realisieren. Es gilt die Beziehung:

$$I_{Soll} = I_0 - G_i * U_a,$$

wobei ein Sollstrom $I_{Soll}$ anhand eines einstellbaren Vorgabestroms $I_0$ der HF- Ausgangsspannung $U_3$ und dem parametrisierbaren Innenleitwert ermittelt wird.

In diesem Zusammenhang ist darauf hinzuweisen, dass der nach wie vor vorhandene Innenwiderstand des Leistungsoszillators keine Rolle mehr spielt und nach außen hin nicht mehr sichtbar ist. Der unterlagerte Spannungsregler regelt den Innenwiderstand des Leistungsoszillators aus. In einem weiteren Anwendungsfall ist das Einstellen eines negativen Widerstands $R_j$ möglich. Hintergrund einer solchen Parametrisierungsvariante ist das Ausregeln eines Kabelwiderstands $R_{Kabel}$ zum Instrument 10 (vgl. z. B. die Leitungen 11, 11'), wenn $R_i$ = $-R_{Kabel}$ ist. Da übliche Messungen von HF-Ausgangsspannung $U_a$ und HF-Ausgangsstrom I3 stets im oder am HF-Generator 21 erfolgen, kann ein Spannungsabfall über das Kabel mit den Leitungen 11, 11' unberücksichtigt bleiben.

Die in der Fig. 3 gezeigten Komponenten können auch als ein Regelkreis verstanden werden, wobei der Soll-Wert anhand der anliegenden Last modifiziert wird. Der Regelkreis gewährleistet ein spannungsgeregeltes Betreiben der Versorgungseinrichtung 20, wobei die HF-Ausgangsspannung U3 (Regelgröße) im Wesentlichen konstant auf oder nahe bei dem Stellwert $U_{Soll1}$ gehalten wird. Die Steuergröße kann die Netzteil-Sollspannung $U_{Soll2}$ oder Netzteil-Istspannung $D_{ist}$ sein. Vorzugsweise wird die Spannung des Leistungsnetzteils oder des Netzteils 22 des HF-Generators variiert. Der Regelkreis kann durch den PI-Regler 23 mit dem zugehörigen Komparator implementiert werden. Die Modifikation des Soll-Werts hängt von der Last ab. Diese Signaleinrichtung wird durch den $R_i$-Simulator implementiert.

Eine erfindungsgemäße Versorgungseinrichtung zur Bereitstellung einer HF- Ausgangsspannung zur Applikation mittels eines elektrochirurgischen Instruments kann umfassen:

- eine HF-Generatoreinheit 21 zur Erzeugung der HF-Ausgangsspannung $U_a$;
- eine Messeinheit zur Bestimmung einer elektrischen Last während der Applikation eines HF-Ausgangsstroms $I_a$;
- eine Signaleinheit zur Ausgabe eines haptisch wahrnehmbaren Signals; und
- eine Steuereinheit 30, die zumindest mit der Messeinheit und der Signaleinheit in kommunikativer Verbindung steht, um die Signaleinheit in Abhängigkeit von der elektrischen Last zu aktivieren.

Bezugszeichenliste

| | |
|---|---|
| 3 | Torso |
| 10 | Instrument 11,11' Leitung |
| 18 | Applikationselektrode |
| 19 | Neutralelektrode |
| 20 | Versorgungseinrichtung |
| 21 | HF-Generator |
| 22 | Netzteil |
| 23 | PI-Regler |
| 24 | Ri-Simulator |
| 25 | Wirkstrom der Recheneinheit |
| 26 | HF-Ausgang |
| 27 | unterlagerter Spannungsregler |
| 29 | Komparator |
| 30 | Steuereinheit |
| $I_o$ | Vorgabestrom |
| $I_a$ | HF-Ausgangsstrom |
| $I_a'$ | Wirkstrom |
| $I_{soll}$ | Sollstrom |
| $U_0$ | HF-Leerlaufspannung |
| $U_a$ | HF-Ausgangsspannung |
| $U_{soll1}$ | Stell wert |
| $U_{soll2}$ | Netzteil-Sollspannung |
| $U_{ist}$ | Netzteil-Istspannung |
| $R_i$ | Widerstand |
| $G_1$ | parametrisierbarer Innenleitwert |
| P | Wirkleistung |

**Patentansprüche**

1. Versorgungseinrichtung zur Bereitstellung einer HF-Ausgangsspannung (U3) zur Applikation mittels eines elektrochirurgischen Instruments (10), umfassend:

   - eine HF-Generatoreinheit (21) zur Erzeugung der HF-Ausgangsspannung
   - eine Messeinheit zur Bestimmung einer elektrischen Last während der Applikation eines HF-Ausgangsstroms (I3);
   - eine Signaleinheit zur Ausgabe eines haptisch wahrnehmbaren Signals; **gekennzeichnet durch**
   - eine Steuereinheit (30), die die Messeinheit und die Signaleinheit umfasst und ausgebildet ist, um die Signaleinheit in Abhängigkeit von der elektrischen Last zu aktivieren.

2. Versorgungseinrichtung nach Anspruch 1, **dadurch gekennzeichnet, dass** die Signaleinheit zur Ausgabe des Signals auf die HF-Generatoreinheit (21) einwirkt, um die HF-Ausgangspannung ($U_3$) und/oder den HF-Ausgangsstrom ($I_a$) zu reduzieren.

3. Versorgungseinrichtung nach Anspruch 1 oder 2, **dadurch gekennzeichnet, dass** die Messeinheit eine Wirkleistungsberecheneinheit (25) zur Berechnung einer Wirkleistung (P) und die Steuereinheit ein Widerstandselement umfasst, um einen gemäß der Wirkleistung (P) angepassten Sollwert ($U_{soll1}$) für die HF- Generatoreinheit (21) vorzugeben.

4. Versorgungseinrichtung nach einem der vorhergehenden Ansprüche, insbesondere nach Anspruch 3, **dadurch gekennzeichnet, dass** das Widerstandselement ($R_1$) einen einstellbaren Widerstand hat.

5. Versorgungseinrichtung nach einem der vorhergehenden Ansprüche, **dadurch gekennzeichnet, dass** die Versorgungseinrichtung zum Betreiben der HF-Generatoreinheit (21) in einer spannungsgeregelten Betriebsart ausgebildet ist, wobei eine Netzteil-Sollspannung ($U_{Soll2}$) eines Leistungsnetzteils (22) variiert wird, um die HF-Ausgangsspannung ($U_a$) im Wesentlichen konstant auf einem Sollwert ($U_{Soll1}$) zu halten.

6. Versorgungseinrichtung nach einem der vorhergehenden Ansprüche, **gekennzeichnet durch** eine Einstellvorrichtung zum Einstellen eines Sollwerts ($U_{Soll1}$), eine Reglereinheit zum Regeln der HF-Generatoreinheit (21) gemäß dem Sollwert ($U_{Soll1}$).

7. Versorgungseinrichtung nach einem der vorhergehenden Ansprüche, **dadurch gekennzeichnet, dass** die Versorgungseinrichtung zur Bestimmung des Sollwerts ($U_{Soll1}$) den HF- Ausgangsstrom ($I_a$) berücksichtigt, insbesondere den Sollwert ($U_{Soll1}$) anhand eines Vorgabewerts ($U_0$) und des HF-Ausgangsstroms ($I_a$) berechnet.

8. Versorgungseinrichtung nach einem der vorhergehenden Ansprüche, insbesondere nach Anspruch 7, **gekennzeichnet durch** eine Komparatoreinheit (29) zur Bestimmung des Sollwerts ($U_{Soll1}$), insbesondere anhand der Formel: $U_{Soll1}=U_0-R_i{}^*I_a$.

9. Versorgungseinrichtung nach einem der vorhergehenden Ansprüche, insbesondere nach Anspruch 6, **dadurch gekennzeichnet, dass** die Reglereinheit einen PI-Regler (23) umfasst.

10. HF-Chirurgiegerät mit einer Versorgungseinrichtung nach einem der vorhergehenden Ansprüche, **gekennzeichnet durch** eine Innenwiderstandeinstellvorrichtung zur Einstellung eines Innenwiderstands, insbesondere gemäß einem Versorgungskabelwiderstand ($R_{Kabel}$) für das elektrochirurgische Instrument (10).

11. HF-Chirurgiegerät nach Anspruch 10, umfassend: ein elektrisches Skalpell oder dergleichen schneidende Instrumente, die an die Versorgungseinrichtung angeschlossen sind.

## Claims

1. Supply device for providing an HF output voltage (U3) for application by means of an electrosurgical instrument (10) comprising:

   • an HF generator unit (21) for generating the HF output voltage
   • a measuring unit for determining an electrical load during application of an HF output current (I3);
   • a signalling unit for emitting a haptically perceptible signal;

   **characterised by**

   • a control unit (30), which comprises the measuring unit and the signalling unit and is configured to activate the signalling unit in dependence on the electrical load.

2. Supply device according to claim 1, **characterised in that** the signalling unit acts to emit the signal to the HF generator unit (21) in order to reduce the HF output voltage ($U_3$) and/or the HF output current ($I_a$).

3. Supply device according to claim I or 2, **characterised in that** the measuring unit comprises an active power calculation unit (25) for calculating an active power (P) and the control unit comprises a resistance element in order to preset a desired value ($U_{des}$) adapted in accordance with the active power (P) for the HF generator unit (21).

4. Supply device according to one of the preceding claims, in particular according to claim 3, **characterised in that** the resistance element ($R_1$) has an adjustable resistance.

5. Supply device according to one of the preceding claims, **characterised in that** the supply device is configured to operate the HF generator unit (21) in a voltage-controlled operating mode, wherein a power unit desired voltage ($U_{des2}$) of a power pack (22) is varied to keep the HF output voltage ($U_a$) substantially constant at a desired value ($U_{des1}$).

6. Supply device according to one of the preceding claims, **characterised by** an adjusting device for adjusting a desired value ($U_{des1}$), a control unit for controlling the HF generator unit (21) in accordance with the desired value ($U_{des1}$).

7. Supply device according to one of the preceding claims, **characterised in that** to determine the desired value ($U_{des1}$) the supply device takes into consideration the HF output current ($I_a$), in particular calculates the desired value ($U_{des1}$) on the basis of a preset value ($U_0$) and the HF output current ($I_a$).

8. Supply device according to one of the preceding claims, in particular according to claim 7, **characterised by** a comparator unit (29) for determining the desired value ($U_{des1}$), in particular on the basis of the formula: $U_{des1} = =U_0-R_i{}^*I_a$.

9. Supply device according to one of the preceding claims, in particular according to claim 6, **characterised in that** the control unit comprises a PI controller (23).

10. HF surgical device with a supply device according to one of the preceding claims, **characterised by** an internal resistance adjusting device for adjusting an internal resistance, in particular in accordance with a supply cable resistance ($R_{cable}$) for the electrosurgical instrument (10).

11. HF surgical device according to claim 10 comprising: an electric scalpel or simular cutting instruments that are connected to the supply device.

## Revendications

1. Dispositif d'alimentation servant à fournir une tension de sortie HF (U3) destinée à être appliquée au moyen d'un instrument électrochirurgical (10), comprenant :

- une unité génératrice HF (21) servant à générer la tension de sortie HF ;
- une unité de mesure servant à déterminer une charge électrique pendant l'application d'un courant de sortie HF (I3) ;
- une unité de signalisation servant à fournir un signal haptiquement perceptible ; **caractérisé par**
- une unité de commande (30) qui comprend l'unité de mesure et l'unité de signalisation et qui est conçue pour activer l'unité de signalisation en fonction de la charge électrique.

2. Dispositif d'alimentation selon la revendication 1, **caractérisé en ce que** l'unité de signalisation servant à fournir le signal agit sur l'unité génératrice HF (21) pour réduire la tension de sortie HF (U3) et/ou le courant de sortie HF ($I_a$).

3. Dispositif d'alimentation selon la revendication l ou 2, **caractérisé en ce que** l'unité de mesure comprend une unité de calcul de puissance active (25) pour calculer une puissance active (P), et l'unité de commande comprend un élément résistif pour prescrire une valeur de consigne ($U_{soll1}$) adapté en fonction de la puissance active (P) pour l'unité génératrice HF (21).

4. Dispositif d'alimentation selon une des revendications précédentes, en particulier selon la revendication 3, **caractérisé en ce que** l'élément résistif ($R_1$) a une résistance réglable.

5. Dispositif d'alimentation selon une des revendications précédentes, **caractérisé en ce que** pour faire fonctionner l'unité génératrice HF (21), le dispositif d'alimentation est conçu avec un mode de fonctionnement régulé en tension qui consiste à faire varier une tension de consigne de bloc d'alimentation ($U_{soll2}$) d'un bloc d'alimentation de puissance (22) pour maintenir la tension de sortie HF ($U_a$) sensiblement constante à une valeur de consigne ($U_{soll1}$).

6. Dispositif d'alimentation selon une des revendications précédentes, **caractérisé par** un dispositif de réglage pour régler une valeur de consigne ($U_{soll1}$) et par une unité de régulation pour réguler l'unité génératrice HF (21) en fonction de la valeur de consigne ($U_{soll1}$).

7. Dispositif d'alimentation selon une des revendications précédentes, **caractérisé en ce que**, pour déterminer la valeur de consigne ($U_{soll1}$), le dispositif d'alimentation tient compte du courant de sortie HF

($I_a$), en particulier calcule la valeur de consigne ($U_{soll1}$) à l'aide d'une valeur standard ($U_0$) et du courant de sortie HF ($I_a$).

8. Dispositif d'alimentation selon une des revendications précédentes, en particulier selon la revendication 7, **caractérisé par** une unité de comparaison (29) pour déterminer la valeur de consigne ($U_{soll1}$), en particulier à l'aide de la formule : $U_{soll1} = U_0 - R_i {}^* I_a$.

9. Dispositif d'alimentation selon une des revendications précédentes, en particulier selon la revendication 6, **caractérisé en ce que** l'unité de régulation comprend un régulateur PI (23).

10. Appareil chirurgical HF pourvu d'un dispositif d'alimentation selon une des revendications précédentes, **caractérisé par** un dispositif de réglage de résistance interne pour régler une résistance interne, en particulier en fonction d'une résistance de câble d'alimentation ($R_{Kabel}$) pour l'instrument électrochirurgical (10).

11. Appareil chirurgical HF selon la revendication 10, comprenant : un scalpel électrique ou des instruments coupants analogues qui sont raccordés au dispositif d'alimentation.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009015278 A